# EUROPEAN PATENT APPLICATION

(11) **EP 3 616 603 A1**
(43) Date of publication of application: **04.03.2020**
(21) Application number: 18191854.1
(22) Date of filing: 30.08.2018
(51) Int. Cl.: A61B 5/00, A61B 5/11, A61B 5/145

(54) **LIGHT BLOCKING STRUCTURE AND METHOD OF BLOCKING AMBIENT LIGHT**

(71) Applicant: RSP Systems A/S, 5260 Odense S (DK)
(72) Inventor: Banke, Stefan Ovesen, 5260 Odense S (DK); Lundsgaard-Nielsen, Signe Maria, 5260 Odense S (DK); Schjoldager Nielsen, Martin, 5260 Odense S (DK)
(74) Representative: Vossius & Partner Patentanwälte Rechtsanwälte mbB

(57) **Abstract**

The present invention relates to an apparatus for transdermal in vivo measurement by Raman spectroscopy at a skin region of a body part and to a method of blocking ambient light. The apparatus comprises at least one housing part, a sensor window provided along said at least one housing part, the window having a first side configured to have the skin region applied thereon, and an optical sensor arranged on a second side of the window. The housing part comprises at least one protruded and/or recessed light blocking structure that extends at least partially around said window and is configured to engage with the skin region.

## Description

The present invention relates to an apparatus for transdermal in vivo measurements by Raman spectroscopy comprising a light blocking structure and a method of blocking ambient light for the preparation of or during such measurements.

### Background

An apparatus for transdermal in vivo measurements by Raman spectroscopy is known, for example, from applicant's co-pending WO 2016/034448 A1. These types of measurements are sensitive and may be distorted by ambient light. There thus continues to be a need in the art to reduce the amount of ambient light reaching the sensor of the apparatus. This is a particular challenge when trying to provide a portable apparatus, which needs to be able to provide reliable and repeatable measurements in various settings.

Different types of light blocking structures have been employed in various fields. For example, US 6 338 240 B2 discloses an optical probe for use in measurements on tissue material of a patient. In order to attenuate ambient light, a light shield is fitted to the probe's housing so as to partially surround the tissue material when it is received in said housing. Another probe adapted to be used with a pulse oximeter is disclosed in US 2007/0027376 A1. The probe comprises a pair of light shielding covers to block ambient light.

EP 2 389 855 A1 discloses a diagnostic camera with a box-shaped attachment for spacing the camera from a surface. Other attachments for dermatoscopes are known from US 9 427 188 B2, WO 2014/134487 A1 and WO 2016/205950 A1. Different types of devices are also known from DE 10 2007 020 078 A1 and US 2013/0220983 A1.

### Summary of the Invention

According to an aspect, an apparatus for transdermal measurements at a skin region of a body part is provided. The apparatus comprises a housing with at least one housing part and an optical sensor as well as a sensor window for said optical sensor provided in said housing part. The apparatus comprises at least one light blocking structure which may extend at least partially around said sensor window and may be protruded and/or recessed. The light blocking structure may be configured to engage with the skin region. The light blocking structure is preferably provided along the at least one housing part.

Furthermore, a method of blocking ambient light for such apparatus is provided, in particular a method for blocking ambient light in preparation for and/or during transdermal measurement at a skin region of a body part, in particular at a skin region of a palm or thenar eminence. The method may comprise (i) applying the body part onto the at least one housing part such that the skin region contacts the housing part at least partially around the light blocking structure, (ii) applying the body part (in particular a palm of a hand) onto the at least one housing part such that the skin region contacts the sensor window, and (iii) engaging the skin region with the light blocking structure to prevent ambient light that propagates between the skin region and the first housing part from reaching the sensor window. As an alternative or in addition to step (iii), a clearance may be formed between the skin region and the housing part, the clearance being formed between the region of contact with the housing part and the region of contact with the sensor window.

The inventors have discovered that unexpected benefits occur when using the light blocking structure according to the invention in transdermal measurements, in particular in very sensitive measurements by Raman spectroscopy. While it is generally assumed that the skin region pressed onto the apparatus around a sensor window will prevent ambient light from reaching the sensor window, it is believed - without wishing to be bound by theory - that ambient light having a low angle of incidence may propagate between the skin region and the surface of the apparatus onto which said skin region is pressed. In other words, it is believed - again without wishing to be bound by theory - that a light guide guiding said low incidence ambient light to the sensor window and sensor may be formed by the skin region in conjunction with the surface of the apparatus onto which said skin region is pressed. The protruded and/or recessed light blocking structures of the present invention have been found to interrupt said light guide in a surprisingly effective manner.

The at least one housing part may comprise at least one skin contacting surface configured to have the skin region applied thereon. Said skin contacting surface may extend at least partially around the light blocking structure, wherein the light blocking structure may be configured to block the ambient light propagating between the skin contacting surface and the skin region. The light blocking structure may be dimensioned such that the skin region, while being engaged with said light blocking structure, contacts the skin contacting surface partially or completely around the light blocking structure and/or such that the skin region contacts the sensor window when the body part is applied onto the apparatus.

The sensor window may have a first side configured to have the skin region applied thereon. The optical sensor may be arranged on a second side of the sensor window. In other words, the sensor window may be positioned between the skin region and the sensor. The apparatus may be configured for a palm of the user, in particular a thenar eminence, or a forearm to be applied and/or pressed onto the housing part and/or onto the sensor window. In other words, the apparatus may be configured to have the palm, in particular the thenar eminence, and/or the forearm at least partially introduced therein and/or applied onto the sensor window.

The sensor window may have a diameter and/or a width of at least 1 mm, at least 3 mm, or at least 5 mm. Alternatively or additionally, the sensor window may have a diameter and/or width of 20 mm or less, 15 mm or less, preferably 10 mm or less.

The sensor window is preferably made from a translucent, more preferably from a transparent material. For example, the sensor window may be made from a magnesium fluoride crystal, which the inventors have found to be particularly useful when performing Raman spectroscopy measurements on skin.

The optical sensor may comprise at least one lens and preferably is a confocal microscopy sensor, in particular a confocal microscopy sensor that comprises at least one light source, optical components defining a light path from said light source to a measurement location, at least one spectrum analysis unit, and optical components defining a return path for Raman scattered light from said measurement location to said spectrum analysis unit. The apparatus and/or the sensor may thus perform spectroscopy measurements, for example measurements by Raman spectroscopy. Light may be emitted from and received on the same side (i.e., the second side) of the sensor window. The measurements may be performed in vivo. The measurements may be performed non-invasively in that they rely on the analysis of light scattered back from the skin region.

The light blocking structure preferably comprises at least one annular segment. In other words, the light blocking structure may comprise one or more rings and/or ring segments forming a partial or complete ring-like structure around the sensor window. The annular segment(s) may be dimensioned such that the skin region, while being engaged with the annular segment(s), contacts the skin contacting surface partially or completely around the light blocking structure and/or such that the skin region contacts the sensor window when the body part is applied onto the apparatus.

The at least one annular segment, in a top view thereof, may form a simple closed curve. Alternatively, the annular segment may have open ends, which may overlap. The light blocking structure may comprise a plurality of separate annular segments that may overlap each other in a circumferential direction.

The at least one annular segment, in a top view thereof, may be shaped as a regular or irregular polygon. The at least one annular segment may also be shaped as an ellipse, oval, circle or spiral. The annular segment may extend around the sensor window over an included angle of at least 180°, at least 270°, or at least 360°. Preferably, the at least one annular segment surrounds the sensor window completely.

In a top view, the at least one annular segment may have a radius of at least 5 mm, preferably at least 10 mm, more preferably at least 15 mm. Alternatively or additionally, the radius may be 30 mm or less, preferably 15 mm or less or 10 mm or less. These shapes and sizes are believed to provide a good tactile feedback to the user, thus allowing the user to perform the measurements at the same location each time the skin region is applied. The at least one annular segment may have a minimum internal width and/or diameter of 10 mm or 20 mm and/or a maximum external width and/or diameter of 30 mm or 20 mm.

A cross section may be taken perpendicular to the sensor window and/or parallel to the central axis of a light beam emitted by the sensor through said sensor window. Such cross section may be perpendicular to the lengthwise extension of the annular segment around the sensor window and/or perpendicular to the skin contacting surface.

In such cross-section, the light blocking structure (in particular, the annular segment or segments) may have a triangular, rectangular, trapezoidal, or rounded protruded and/or recessed shape.

Alternatively or additionally, the light blocking structure (in particular, the annular segment or segments) in such cross section may have a width of at least 1 mm, preferably of at least 2 mm, more preferably of at least 3 mm. Said width may be measured at a base of the light blocking structure and/or as the light blocking structure's lateral extent measured parallel to the skin contacting surface. The width may be 6 mm or less, preferably 4 mm or less, more preferably 3 mm or less.

Alternatively or additionally, when the light blocking structure (in particular, the annular segment or segments) is protruded, it may have a height of at least 1 mm, preferably of at least 2 mm in such cross section (e.g., it may protrude by this distance over the skin contacting surface and/or the sensor window). The height may be 5 mm or less, preferably 3 mm or less. When the light blocking structure (in particular, the annular segment or segments) is recessed, it may have a depth of at least 1 mm, preferably of at least 2 mm in such cross section (e.g., a bottom thereof may be recessed by this distance from the skin contacting surface and/or the sensor window). The depth may be 5 mm or less, preferably 3 mm or less.

Protruded and recessed light blocking structures may be combined. In particular, protruded and recessed annular segments and/or rings may be combined to form the light blocking structure. For example, the light blocking structure may include a first annular segment or ring that is protruded and a second annular segment or ring that is recessed. The second annular segment may extend at least partially or completely around the first annular segment. For example, the second annular segment may extend around the first annular segment between said first annular segment and the skin contacting surface of the housing part. With such configuration, the first annular segment and/or the second annular segment may engage with the skin surface. For example, only the first annular segment may provide the engagement in certain configurations.

The housing part may comprise at least one band of light absorbing material. The at least one band may extend at least partially or completely around the at least one light blocking structure. In particular, the band may at least partially or completely surround the at least one annular segment. The band may be at least 2 mm wide, preferably at least 5 mm.

The band may be integrally formed with the at least one light blocking structure, in particular with the at least one annular segment. In other words, the at least one band and the light blocking structure (in particular, the annular segment(s)) may be formed as a unitary component. This unitary component may be fixedly attached to the at least one housing part.

The light blocking structure, the at least one annular segment and/or the at least one band may be made from a thermoplastic elastomer, a thermoplastic vulcanizate, and/or an elastomer. For example, the light blocking structure, the at least one annular segment and/or the at least one band may be made from a material comprising cured rubber particles in a thermoplastic phase. One such material is Santoprene, which comprises ethylene propylene diene monomer rubber particles in a polypropylene matrix. However, also other materials, for example neoprene, have been found to provide the desired results. It is believed that these materials allow for a reliable engagement with the skin region and blocking of light. The light blocking structure, the at least one annular segment and/or the at least one band may be made from a material which is different from that of the housing part.

The material from which the light blocking structure, the at least one annular segment and/or the at least one band is made is preferably a dark material, more preferably a black material. The annular segment(s) may be fixedly attached to and/or co-molded with the at least one housing part. Alternatively or additionally, the material from which the light blocking structure, the at least one annular segment and/or the at least one band is made may be a material having a transmittance T of less than 10%, preferably less than 5% or less than 2%, in the range of 700 to 1100 nm. The transmittance may be measured on a sheet of material having a thickness of 1 mm (i.e., by measuring the transmittance parallel to and/or across the thickness direction).

Further to the above-mentioned light blocking structure, the apparatus may also comprise a light shield configured to at least partially cover the body part (e.g., the hand, palm or thenar eminence) applied onto the sensor window. While such light shield alone might not be sufficient for blocking ambient light to a sufficient degree for performing reliable and repeatable measurements (as mentioned above, and without wanting to be bound by theory, because it is believed that ambient light with a low angle of incidence may be guided to the sensor window despite the presence of such light shield via a light guide formed between the skin region and the surface of the apparatus onto which said skin region is applied), such light shield may reduce the amount of ambient light reaching the skin surface and/or the amount of ambient light passing through the skin and/or the body part itself.

The light shield may be movable. In particular, the light shield may be movable from at least one closed position to at least one open position. For example, the light shield may be pivotally connected to the housing (e.g., it may be attached to the housing via a hinge).

In the at least one closed position, the light shield may cover the sensor window and/or seal around the sensor window such that the sensor window is protected from dust and/or humidity (or other environmental influences). This may be particularly advantageous when the apparatus is portable.

In the at least one open position, the light shield may be configured to allow insertion of the at least one body part so that the skin region can be applied onto the sensor window and engaged with the light blocking structure.

The light shield may be configured to seal around the body part. In particular, the light shield may be configured to seal around the hand, thumb, wrist or thenar eminence of the user. Such seal may be provided in the open position. Alternatively or additionally, the light shield may be movable into at least one intermediate position for this purpose.

In order to improve the seal with the housing and/or with the body part, the light shield may comprise one or more sealing lips. These sealing lips may be made from a flexible material, e.g. a material which is more flexible than that of the light shield itself and/or than that of the housing part. Preferably, the light shield and/or the sealing lip(s) are made from a dark material, more preferably from a black material. At least one of the sealing lips may be provided with a first cut out or recess, in particular with a cut out configured to accomodate the user's thumb and/or wrist. Additionally (or also alternatively) a second cut out may be provided to accommodate one or more of the user's fingers.

In the closed position, the sealing lip(s) may contact the housing (e.g., the at least one housing part) and may seal therewith in order to protect the sensor window.

The apparatus may be portable. The apparatus may weight less than 2 kg, preferably less than 1 kg, more preferably less than 500 g. The apparatus may be less than 40 cm, preferably less than 30 cm high, wide and deep. The apparatus may have a battery and/or may not require an external electrical connection for providing measurements.

The apparatus may be particularly configured and/or the method may be used for transdermal in vivo measurement of different analytes. Exemplary analytes include glucose, lactate, haemoglobin, cholesterol, alcohol, urea and/or one or more drugs (e.g., medicaments, and/or prohibited substances). Raman spectroscopy is preferred, but also other methods, in particular other spectroscopy measurement methods, might be employed (depending on the particular analyte).

### Brief Description of the Drawings

The invention will be described in more detail with reference to the figures below. These figures disclose embodiments of the invention for illustrational purposes only. In particular, the disclosure provided by the figures is not meant to limit the scope of protection conferred by the invention. The figures illustrate:
- **Fig. 1**: A schematic perspective view of an apparatus for transdermal in vivo measurement by Raman spectroscopy according to a preferred embodiment of the invention in a closed position.
- **Fig. 2**: A schematic perspective view of the apparatus of Fig. 1 in an open position.
- **Fig. 3**: A schematic perspective view of the apparatus of Figs. 1 and 2 with a body part inserted therein.
- **Fig. 4**: A detailed view of the light blocking structure used in the apparatus according to Figs. 1, 2 and 3.
- **Fig. 5**: A schematic cross sectional view of the light blocking structure shown in Fig. 4.
- **Fig. 6**: A schematic top view of an alternative light blocking structure according to the invention.

### Detailed Description

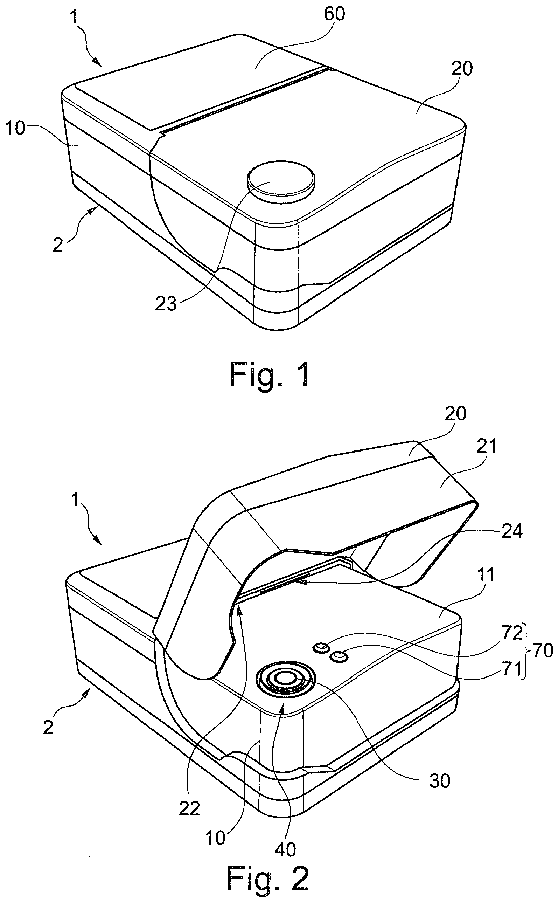

Figs. 1, 2 and 3 illustrate an exemplary apparatus 1 according to the present invention. The apparatus preferably serves for measuring a quantity or concentration of an analyte through the skin, preferably in vivo. Such transdermal measurement is preferably performed by Raman spectroscopy.

As shown, the apparatus 1 may comprise a housing 2, which may at least partially be covered or closed by an optional light shield 20. The light shield 20 may also be referred to as a lid. The light shield 20 may be connected to the housing 2 via a hinge 24.

The apparatus 1 may further comprise a sensor arrangement, preferably a sensor window 30. The sensor arrangement or sensor window 30 may be located along a housing part 10, in particular along a skin contacting surface 11 of the housing part 10 that is configured to have a skin region of a user applied thereon.

The apparatus 1 may further comprise a light blocking structure 40. The light blocking structure 40 is preferably arranged along said housing part 10, in particular along said skin contacting surface 11. As shown in Fig. 2, the skin contacting surface 11 may extend around and/or surround the light blocking structure 40 (e.g., surrounds it completely). The light blocking structure 40 preferably is arranged proximate the sensor window 30, more preferably such that the light blocking structure 40 extends at least partially around and/or surrounds said sensor window 30 (e.g., surrounds it completely). When using the apparatus 1, the user preferably arranges the body part on which the measurement is to be performed on the skin contacting surface 11 such that the skin region contacts the skin contacting surface 11, is engaged with the light blocking structure 40, and contacts the sensor window 30.

The apparatus 1 may further comprise a first user interface 60, which may be arranged along an outer portion of the housing 2, and/or a second user interface 70, which may be arranged such that it is covered by the light shield 20 when the light shield is in a closed position.

The first user interface 60 may, for example, be configured as or include a screen (e.g., a touch screen) on which the results of a measurement can be displayed and/or which may be used to control the settings of the apparatus 1. Alternatively or additionally, the results could also be displayed on a remote and/or hand-held device, such as for example a smartphone. Such remote and/or hand-held device could also be used to control the apparatus 1.

The second user interface 70 may be configured as one or more touch control elements, such as one or more buttons 71, 72. Such user interface 70 that is covered by the light shield 20 may, for example, serve to initiate a measurement by the user when a hand, finger or thumb is inserted into the apparatus 1.

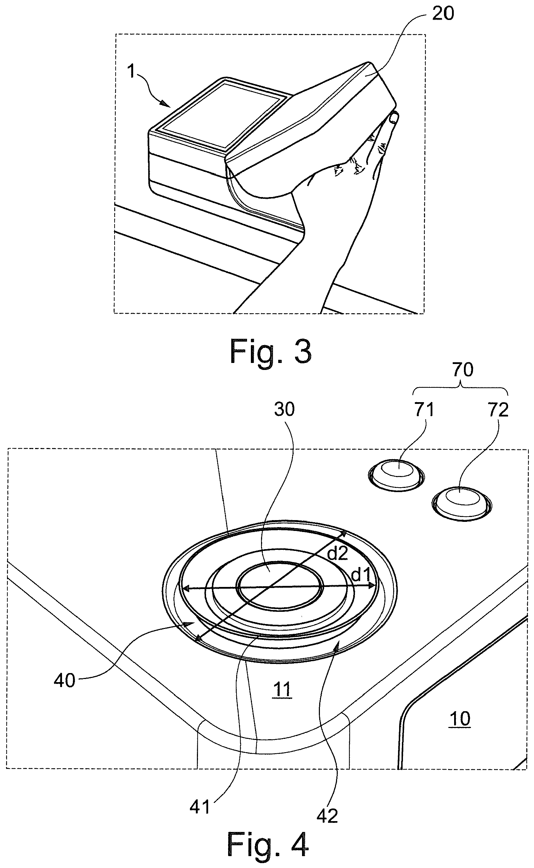

As shown in Fig. 2, the light shield 20 may comprise a lip 21, which may be flexible. The lip 21 may serve to seal with the housing 2 when the light shield 20 is closed, thereby minimizing the amount of dust, water and/or humidity entering into the portion of the apparatus covered by the light shield 20. The lip 21 be provided with a cut out 22 that is configured to accommodate and/or seal around a portion of the user's body part when inserted into the apparatus, for example the user's thumb or wrist (see Fig. 3).

Fig. 4 is a detailed view of the light blocking structure 40 and the second user interface 70. As shown, the light blocking structure 40 may comprise a protruded structure 41 (which, as shown in the exemplary embodiment of Fig. 3, may be a protruded annular segment, in particular a protruded ring) which protrudes over the skin contacting surface 11. Alternatively or additionally, a recessed structure 42 (which, as shown in the exemplary embodiment of Fig. 3, may be a recessed annular segment, in particular a recessed ring) which is recessed from the skin contacting surface 11 may be provided. When both a protruded structure 41 and a recessed structure 42 are present, it may be sufficient for the skin region to be engaged with only one of these structures, for example only with the protruded structure 41.

The protruded structure 41 has an internal width or diameter d1 while the recessed structure 42 has an internal width or diameter d2. The diameter d1 and the diameter d2 are preferably larger than 10 mm (more preferably larger than 20 mm) and/or smaller than 40 mm (more preferably smaller than 30 mm).

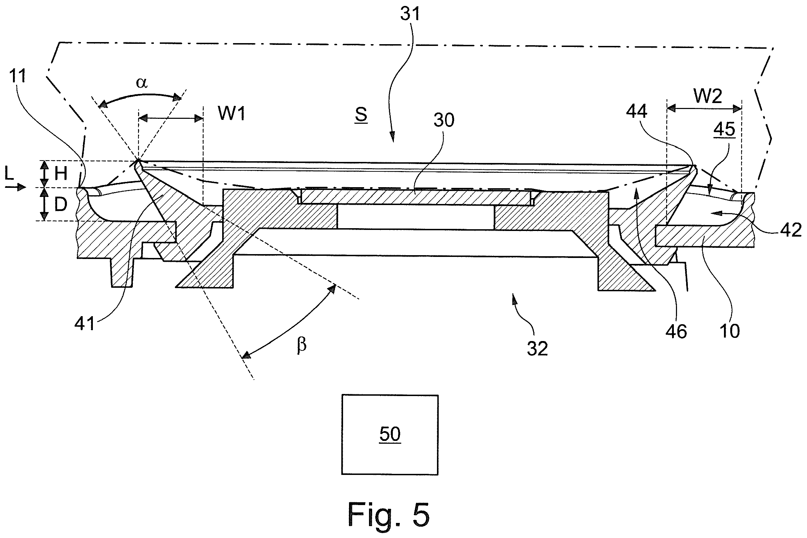

Fig. 5 schematically illustrates a cross section perpendicular to the sensor window and/or parallel to the central axis of a light beam emitted through said sensor window 30 by a sensor 50 (e.g., a Raman sensor arrangement) when performing the measurement. As shown, the skin region S may be applied onto the housing part 10 such that it contacts the skin contacting surface 11 and the sensor window 30. The skin region S preferably contacts the sensor window 30 along a first side 31 thereof. The first side 31 preferably is opposite a second side 32 of the sensor window 30 on which the sensor 50 may be located. The second side 32 may be interior to the housing 2.

While it would have been assumed that the skin region S pressed onto the skin contacting surface 11 around the sensor window 30 would prevent ambient light from reaching the window 30 (in particular, if the body part on which the measurement is performed is additionally covered by a light shield), it has been found by the inventors that the measurement results may still be distorted in an undesirable manner under some circumstances. Without wishing to be bound by theory, it is believed that the skin region S pressed onto the skin contacting surface 11 surprisingly functions as a wave guide for ambient light L with a low angle of incidence, which thus propagates between the skin region S and the skin contacting surface 11 towards the sensor window 30.

The light blocking structure 40 may inhibit this effect by contacting the skin region S with the protruded ring 41, whereby the light guide is believed to be interrupted. Preferably, the protruded ring 41 is pressed into and deforms the skin surface S to some extent (see Fig. 5). For this purpose, a pointed tip or rim 44 may be provided at the upper end of the light blocking structure 40 to contact the skin region S. As shown in the cross sectional view of Fig. 5, said rim 44 may form an acute internal angle α. The angle α preferably is smaller than 90°, more preferably smaller than 60°. The tip or rim 44 may me pointed. It may be pointed upwards.

The protruded structure 41 may be provided with a generally triangular cross section in this context (see Fig. 5), but could also have a rectangular, trapezoidal, rounded or oval shape. The generally triangular cross section may protrude with an included angle β of smaller than 90°, preferably smaller than 45°.

The recessed structure 42 may or may not be configured to contact the skin region S. In both cases the light guide may be interrupted. For example, when the recessed structure 42 is configured to contact the skin region S (not shown in Fig. 5), the skin region S may be deformed such that the light transmitted along the skin region S is minimized and/or edges of the recessed structure 42 may press into the skin region S such that the light guide is interrupted. Alternatively or additionally, the recessed structure 42 (and/or the protruding structure 41) may be configured to create a clearance 45 between the skin region S and the skin contacting surface 11 in which the skin region S does not contact the housing part 10 when it is arranged thereon to perform the measurement. It is assumed that also such clearance interrupts the light guide formed between the skin region S and the skin contacting surface 11 because the fraction of light reaching the clearance 45 will be scattered and mostly attenuated in said clearance 45.

As illustrated in Fig. 5, the light blocking structure may in some cases be configured to interrupt the light guide by providing several of these effects in conjunction, for example an engagement of the skin region S with the protruded structure 41 and, in addition, the clearance 45 along the recessed structure 42.

As further illustrated in Fig. 5, the protruded structure 41 may have a width W1 while the recessed structure 42 may have a width W2 as measured parallel to the skin contacting surface 11 from the tip or rim 44 to the radially innermost end of the protruded structure 41. The widths W1 and W2 are preferably at least 1 mm, at least 2 mm, or at least 3 mm and/or 6 mm or less, 4 mm or less, or 3 mm or less.

Alternatively or additionally, the protruded structure 41 may have a height H as measured from the skin contacting surface 11 of at least 1 mm or at least 2 mm and/or 5 mm or less or 3 mm or less. The recessed structure 42 may have a depth D as measured from the skin contacting surface 11 of at least 1 mm or at least 2 mm and/or 5 mm or less or 3 mm or less.

While Fig. 5 illustrates the cross section of the protruded structure 41 and the recessed structure 42 for the light blocking structure 40 according to Fig. 4 (where the structures 41, 42 are provided as closed rings), such cross sectional shapes may be provided also for any other protruded and/or recessed light blocking structures (in particular, for any protruded and/or recessed annular segment(s)) according to the present invention.

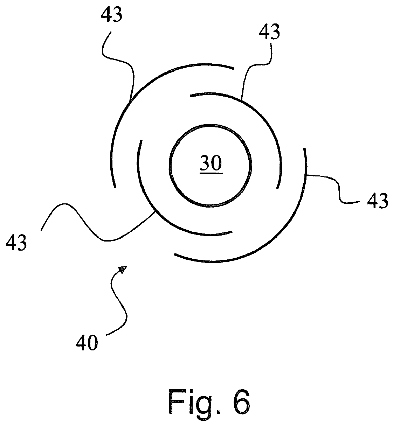

One such alternative light blocking structure 40 is schematically illustrated in Fig. 6. In this case, the light blocking structure 40 comprises a series of overlapping, preferably annular segments 43 arranged around the sensor window 30. As will be evident to the skilled person from the present disclosure, these segments 43 may equally interrupt the light guide formed between the skin region and the skin contacting surface, thereby preventing ambient light with a low incident angle from reaching the sensor window 30.

The light shield 20 may include a recess 23 to accommodate the light blocking structure when the light shield 20 is in the closed position (see Fig. 1).

While aspects and embodiments of the invention are illustrated and described in detail in the figures and in the foregoing description, such illustration and description is to be considered illustrative or exemplary and not restrictive. Also reference signs in the claims should not be construed as limiting the scope.

It will also be understood that changes and modifications may be made by those of ordinary skill within the scope and spirit of the following claims. In particular, the present invention covers further embodiments with any combination of features from different aspects or embodiments described above. It is also to be noted in this context that the invention covers all further features shown in the figures individually, although they may not have been described in the previous. Also, single alternatives of the embodiments described in the figures and the description and single alternatives of features thereof can be disclaimed from the subject matter according to aspects of the invention.

Whenever the word "comprising" is used in the claims, it should not be construed to exclude other elements or steps. Similarly, the indefinite article "a" or "an" does not exclude a plurality. A single unit may fulfill the functions of several features recited in the claims. It should also be understood that the terms "essentially", "substantially", "about", "approximately" and the like used in connection with an attribute or a value may define the attribute or the value in an exact manner in the context of the present disclosure. The terms "essentially", "substantially", "about", "approximately" and the like could thus also be omitted when referring to the respective attribute or value.

The present invention relates inter alia to the following aspects:
1. An apparatus for transdermal in vivo measurement by Raman spectroscopy at a skin region of a body part, the apparatus comprising:
   a housing with at least one housing part;
   a sensor window provided along said at least one housing part, the window having a first side configured to have the skin region applied thereon; and
   an optical sensor arranged on a second side of the window;
   wherein the housing part comprises at least one protruded and/or recessed light blocking structure configured to engage with the skin region, wherein the light blocking structure extends at least partially around said window.
2. The apparatus of aspect 1, wherein the window is configured to have a palm of a hand of the user applied thereon, more preferably a thenar eminence.
3. The apparatus according to aspect 1 or 2, wherein the at least one housing part comprises at least one skin contacting surface configured to have the skin region applied thereon, wherein the skin contacting surface extends at least partially around the light blocking structure, and wherein the light blocking structure is configured to block ambient light propagating between the skin contacting surface and the skin region.
4. The apparatus according to aspect 1, 2 or 3, wherein the light blocking structure comprises at least one annular segment, preferably wherein the at least one annular segment extends around the sensor window over an included angle of 180° or more, 270° or more, or 360° or more.
5. The apparatus according to the preceding aspect, wherein the at least one annular segment surrounds the window completely.
6. The apparatus according to any of aspects 3 to 5, wherein the light blocking structure, in particular the at least one annular segment, is dimensioned such that the skin region contacts the skin contacting surface at least partially around the light blocking structure.
7. The apparatus according to any one of aspects 4 to 6, wherein the at least one annular segment has a radius of at least 5 mm, preferably at least 10 mm, more preferably at least 15 mm.
8. The apparatus according to any one of aspects 4 to 7, wherein the at least one annular segment has a radius of 40 mm or less, preferably 30 mm or less.
9. The apparatus according to any one of aspects 4 to 8, wherein the at least one annular segment, in a cross section perpendicular to a lengthwise extension thereof, has a width of at least 1 mm, preferably of at least 2 mm, more preferably of at least 3 mm.
10. The apparatus according to any one of aspects 4 to 9, wherein the at least one annular segment, in a cross section perpendicular to the lengthwise extension thereof, has a width of 6 mm or less, preferably of 4 mm or less, more preferably of 3 mm or less.
11. The apparatus according to any one of aspects 4 to 10, wherein the at least one annular segment protrudes and, in a cross section perpendicular to the lengthwise extension thereof, has a height of at least 1 mm, preferably of at least 2 mm, and/or wherein the at least one annular segment is recessed and, in a cross section perpendicular to the lengthwise extension thereof, has a depth of at least 1 mm, preferably of at least 2 mm.
12. The apparatus according to any one of aspects 4 to 11, wherein the at least one annular segment protrudes and, in a cross section perpendicular to the lengthwise extension thereof, has a height of 5 mm or less, preferably a height of 3 mm or less, and/or wherein the at least one annular segment is recessed and, in a cross section perpendicular to the lengthwise extension thereof, has a depth of 5 mm or less, preferably a depth of 3 mm or less.
13. The apparatus according to any one of aspects 4 to 12, wherein the at least one annular segment has a triangular, rectangular, trapezoidal, or rounded cross-sectional shape.
14. The apparatus according to any one of aspects 4 to 13, wherein the at least one annular segment, in a top view thereof, forms an ellipse, oval, circle or spiral, or is shaped as a regular or irregular polygon.
15. The apparatus according to any one of aspects 4 to 14, wherein, in a top view thereof, the at least one annular segment forms a simple closed curve and/or wherein circumferential ends of the at least one annular segment overlap.
16. The apparatus according to any one of the preceding aspects, wherein the light blocking structure comprises a plurality of separate annular segments that overlap each other in a circumferential direction.
17. The apparatus according to any one of the preceding aspects, wherein the housing part comprises at least one band of light absorbing material, wherein the at least one band extends at least partially around the at least one light blocking structure, preferably at least partially around the at least one annular segment.
18. The apparatus according to the preceding aspect, wherein the at least one band is at least 2 mm, preferably at least 5 mm wide.
19. The apparatus according to aspect 17 or 18, wherein the at least one band is integrally formed with the at least one light blocking structure, preferably integrally formed with the at least one annular segment.
20. The apparatus according to the preceding aspect, wherein the at least one band and the at least one annular segment are formed as a unitary component that is fixedly attached to the at least one housing part.
21. The apparatus according to any one of the preceding aspects, wherein the light blocking structure, the at least one annular segment and/or the at least one band is/are made from a dark material, preferably a black material.
22. The apparatus according to any one of the preceding aspects, wherein the light blocking structure, the at least one annular segment and/or the at least one band is/are made from a thermoplastic elastomer, a thermoplastic vulcanizate, and/or an elastomer.
23. The apparatus according to any one of the preceding aspects, wherein the light blocking structure, the at least one annular segment and/or the at least one band is/are made from made from a material comprising cured rubber particles, preferably ethylene propylene diene monomer rubber, in a thermoplastic phase, preferably polypropylene.
24. The apparatus according to the preceding aspect, wherein the material is Santoprene.
25. The apparatus according to any one of the preceding aspects, wherein the light blocking structure, the at least one annular segment and/or the at least one band is/are made from neoprene.
26. The apparatus according to any one of the preceding aspects, wherein the apparatus further comprises a light shield configured to at least partially cover the body part applied onto the sensor window.
27. The apparatus according to the preceding aspect, wherein the light shield is movable into at least one open position configured to allow insertion of the body part, and at least one closed position.
28. The apparatus according to the preceding aspect, wherein the light shield covers the window when in said closed position.
29. The apparatus according to aspect 26, 27, or 28, wherein the light shield is movable into at least one intermediate position, wherein the light shield is configured to seal around the body part when in said intermediate position.
30. The apparatus according to any one of aspects 26 to 29, wherein the light shield is pivotally mounted to the housing.
31. The apparatus according to any one of aspects 26 to 30, wherein the light shield comprises one or more flexible lips configured to seal around the body part.
32. The apparatus according to the preceding aspect, wherein the light shield comprises at least one flexible lip with a cut out for the user's thumb and/or wrist.
33. The apparatus according to aspect 31 or 32, wherein, when the light shield is in the closed position, the at least one flexible lip is configured to seal with the at least one housing part such that the window is protected from environmental influences.
34. The apparatus according to aspect 31, 32 or 33, wherein the at least one flexible lip is made from a dark material, preferably from a black material.
35. The apparatus according to any one of the preceding aspects, wherein the window has a diameter and/or a width of at least 1 mm, at least 3 mm, or at least 5 mm.
36. The apparatus according to any one of the preceding aspects, wherein the window has a diameter and/or width of 20 mm or less, 15 mm or less, preferably 10 mm or less.
37. The apparatus according to any one of the preceding aspects, wherein the window is made from a transparent material.
38. The apparatus according to any one of the preceding aspects, wherein the window is made from a magnesium fluoride crystal.
39. The apparatus according to any one of the preceding aspects, wherein the optical sensor comprises at least one lens, preferably wherein the optical sensor is a confocal microscopy sensor, more preferably a confocal microscopy sensor for Raman spectroscopy comprising a light source, optical components defining a light path from said light source to a measurement location, a spectrum analysis unit, and optical components defining a return path for Raman scattered light from said measurement location to said spectrum analysis unit.
40. The apparatus according to any one of the preceding aspects, wherein the apparatus is an apparatus for transdermal in vivo measurement of glucose, lactate, haemoglobin, cholesterol, alcohol, urea and/or drug.
41. A method of blocking ambient light for a transdermal measurement by Raman spectroscopy, the method comprising:
   - providing an apparatus according to any of the preceding aspects;
   - applying the body part to the at least one housing part such that the skin region contacts the housing part at least partially around the light blocking structure;
   - engaging the skin region with the light blocking structure to prevent ambient light that propagates between the skin region and the first housing part from reaching the sensor window.

## Claims

1. An apparatus (1) for transdermal in vivo measurement by Raman spectroscopy at a skin region (S) of a body part, the apparatus (1) comprising:
a housing (2) with at least one housing part (10);
a sensor window (30) provided along said at least one housing part (10), the sensor window (30) having a first side (31) configured to have the skin region (S) applied thereon; and
an optical sensor (50) arranged on a second side (32) of the window (30);
wherein the housing part (10) comprises at least one protruded and/or recessed light blocking structure (40) that extends at least partially around said window (30) and is configured to engage with the skin region (S).

2. The apparatus (1) of claim 1, wherein the window (30) is configured to have a palm of a hand of the user applied thereon, more preferably a thenar eminence.

3. The apparatus (1) according to claim 1 or 2, wherein the at least one housing part (10) comprises at least one skin contacting surface (11) configured to have the skin region (S) applied thereon, wherein the skin contacting surface (11) extends at least partially around the light blocking structure (40), and wherein the light blocking structure (40) is configured to block ambient light (L) propagating between the skin contacting surface (11) and the skin region (S).

4. The apparatus (1) according to claim 3, wherein the light blocking structure is dimensioned such that the skin region (S) contacts the skin contacting surface (11) at least partially around the light blocking structure (40) as well as along the sensor window (30).

5. The apparatus (1) according to any one of the preceding claims, wherein the light blocking structure (40) comprises at least one annular segment (41, 42, 43), preferably wherein the at least one annular segment (41, 42) extends around the sensor window (30) over an included angle of 180° or more, 270° or more, or 360° or more.

6. The apparatus (1) according to claim 5, wherein the at least one annular segment (41, 42) surrounds the sensor window (30) completely.

7. The apparatus (1) according to claim 5 or 6,
wherein the at least one annular segment (41) is protruded with respect to the skin contacting surface (11);
wherein, in a cross section perpendicular to the lengthwise extension thereof, the at least one annular segment (41) has a height (H) of at least 1 mm, preferably of at least 2 mm; and
wherein, in a cross section perpendicular to the lengthwise extension thereof, the at least one annular segment (41) has a height (H) of 5 mm or less, preferably a height of 3 mm or less.

8. The apparatus (1) according to claim 5, 6 or 7,
wherein the at least one annular segment (41, 42), in a top view thereof, forms an ellipse, oval, circle or spiral, or is shaped as a regular or irregular polygon, and/or
wherein the light blocking structure (40) comprises a plurality of separate annular segments (43) that overlap each other in a circumferential direction.

9. The apparatus (1) according to any one of the preceding claims, wherein the light blocking structure (40) is made from a dark material, preferably a black material.

10. The apparatus (1) according to any one of the preceding claims, wherein the light blocking structure (40) is made from a thermoplastic elastomer, a thermoplastic vulcanizate, and/or an elastomer.

11. The apparatus (1) according to any one of the preceding claims,
wherein the apparatus (1) further comprises a light shield (20) configured to at least partially cover the body part applied onto the sensor window (30),
preferably wherein the light shield (20) is pivotally mounted to the housing and movable into at least one open position configured to allow insertion of the body part, and at least one closed position, wherein the light shield (20) covers the sensor window (30) when in said closed position.

12. The apparatus (1) according to claim 11, wherein the light shield (20) comprises one or more flexible lips (21) configured to contact and seal around the body part, in particular around the user's thumb and/or wrist.

13. The apparatus (1) according to any one of the preceding claims, wherein the apparatus (1) is an apparatus (1) for transdermal in vivo measurement of glucose

14. The apparatus (1) according to any one of the preceding aspects, wherein the apparatus (1) is an apparatus (1) for transdermal in vivo measurement of lactate, haemoglobin, cholesterol, alcohol, urea and/or drug.

15. A method of blocking ambient light for a transdermal measurement by Raman spectroscopy, the method comprising:
- providing an apparatus (1) according to any of the preceding claims;
- applying the body part to the housing part (10) such that the skin region (S) contacts the housing part (10) at least partially around the light blocking structure (40);
- engaging the skin region (S) with the light blocking structure (40) to prevent ambient light (L) that propagates between the skin region (S) and the housing part (10) from reaching the sensor window (30).
